# EUROPÄISCHE PATENTSCHRIFT

(11) **EP 3 044 302 B1**
(45) Veröffentlichungstag und Bekanntmachung des Hinweises auf die Patenterteilung: **25.10.2017**
(21) Anmeldenummer: 14744019.2
(22) Anmeldetag: 18.07.2014
(51) Int. Cl.: C11D 3/386, C12N 9/54, C12N 9/50

(54) **FESTES TEXTILWASCHMITTEL MIT VERBESSERTER PROTEASELEISTUNG**
SOLID TEXTILE DETERGENT WITH IMPROVED PROTEASE PERFORMANCE
DÉTERGENT SOLIDE À ACTIVITÉ PROTÉASIQUE AMÉLIORÉE POUR TEXTILES

(30) Priorität: 12.09.2013 DE 102013218253
(43) Veröffentlichungstag der Anmeldung: 20.07.2016
(73) Patentinhaber: Henkel AG & Co. KGaA, 40589 Düsseldorf (DE)
(72) Erfinder: O'CONNELL, Timothy, 40547 Düsseldorf (DE); TONDERA, Susanne, 40597 Düsseldorf (DE); HELLMUTH, Hendrik, 40237 Düsseldorf (DE); WEBER, Thomas, 41541 Dormagen (DE); MUSSMANN, Nina, 47877 Willich (DE)
(74) Vertreter: Strohe-Kamp, Geertje
(86) Internationale Anmeldenummer: PCT/EP2014/065470
(87) Internationale Veröffentlichungsnummer: WO 2015/036152

(56) Entgegenhaltungen:
- WO-A1-95/23221
- WO-A1-2013/087545
- DE-T2- 69 535 736

### SEQUENCE LISTING

<110> Henkel AG & Co. KGaA
<120> Festes Textilwaschmittel mit verbesserter Proteaseleistung
<130> PT032002
<150> DE102013218253.8
   <151> 2013-09-12
<160> 2
<170> PatentIn version 3.5
<210> 1
   <211> 269
   <212> PRT
   <213> Bacillus lentus
<400> 1
   Ala Gln Ser Val Pro Trp Gly Ile Ser Arg Val Gln Ala Pro Ala Ala
   1               5                   10                  15
   His Asn Arg Gly Leu Thr Gly Ser Gly Val Lys Val Ala Val Leu Asp
               20                  25                  30
   Thr Gly Ile Ser Thr His Pro Asp Leu Asn Ile Arg Gly Gly Ala Ser
           35                  40                  45
   Phe Val Pro Gly Glu Pro Ser Thr Gln Asp Gly Asn Gly His Gly Thr
       50                  55                  60
   His Val Ala Gly Thr Ile Ala Ala Leu Asn Asn Ser Ile Gly Val Leu
   65                  70                  75                  80
   Gly Val Ala Pro Ser Ala Glu Leu Tyr Ala Val Lys Val Leu Gly Ala
                   85                  90                  95
   Asp Gly Arg Gly Ala Ile Ser Ser Ile Ala Gln Gly Leu Glu Trp Ala
               100                 105                 110
   Gly Asn Asn Gly Met His Val Ala Asn Leu Ser Leu Gly Ser Pro Ser
           115                 120                 125
   Pro Ser Ala Thr Leu Glu Gln Ala Val Asn Ser Ala Thr Ser Arg Gly
       130                 135                 140
   Val Leu Val Val Ala Ala Ser Gly Asn Ser Gly Ala Ser Ser Ile Ser
   145                 150                 155                 160
   Tyr Pro Ala Arg Tyr Ala Asn Ala Met Ala Val Gly Ala Thr Asp Gln
   165 170 175
   Asn Asn Asn Arg Ala Ser Phe Ser Gln Tyr Gly Ala Gly Leu Asp Ile
   180 185 190
   Val Ala Pro Gly Val Asn Val Gln Ser Thr Tyr Pro Gly Ser Thr Tyr
   195 200 205
   Ala Ser Leu Asn Gly Thr Ser Met Ala Thr Pro His Val Ala Gly Ala
   210 215 220
   Ala Ala Leu Val Lys Gln Lys Asn Pro Ser Trp Ser Asn Val Gln Ile
   225 230 235 240
   Arg Asn His Leu Lys Asn Thr Ala Thr Ser Leu Gly Ser Thr Asn Leu
   245 250 255
   Tyr Gly Ser Gly Leu Val Asn Ala Glu Ala Ala Thr Arg
   260 265
<210> 2
   <211> 269
   <212> PRT
   <213> Bacillus lentus
<400> 2
   Ala Gln Ser Val Pro Trp Gly Ile Ser Arg Val Gln Ala Pro Ala Ala
   1               5                   10                  15
   His Asn Arg Gly Leu Thr Gly Ser Gly Val Lys Val Ala Val Leu Asp
               20                  25                  30
   Thr Gly Ile Ser Thr His Pro Asp Leu Asn Ile Arg Gly Gly Ala Ser
           35                  40                  45
   Phe Val Pro Gly Glu Pro Ser Thr Gln Asp Gly Asn Gly His Gly Thr
       50                  55                  60
   His Val Ala Gly Thr Ile Ala Ala Leu Asn Asn Ser Ile Gly Val Leu
   65                  70                  75                  80
   Gly Val Ala Pro Ser Ala Glu Leu Tyr Ala Val Lys Val Leu Gly Ala
                   85                  90                  95
   Asp Gly Glu Gly Ala Ile Ser Ser Ile Ala Gln Gly Leu Glu Trp Ala
               100                 105                 110
   Gly Asn Asn Gly Met His Val Ala Asn Leu Ser Leu Gly Ser Pro Ser
               115                 120                 125
   Pro Ser Ala Thr Leu Glu Gln Ala Val Asn Ser Ala Thr Ser Arg Gly
       130                 135                 140
   Val Leu Val Val Ala Ala Ser Gly Asn Ser Gly Ala Ser Ser Ile Ser
   145                 150                 155                 160
   Tyr Pro Ala Arg Tyr Ala Asn Ala Met Ala Val Gly Ala Thr Asp Gln
                   165                 170                 175
   Asn Asn Asn Arg Ala Ser Phe Ser Gln Tyr Gly Ala Gly Leu Asp Ile
               180                 185                 190
   Val Ala Pro Gly Val Asn Val Gln Ser Thr Tyr Pro Gly Ser Thr Tyr
           195                 200                 205
   Ala Ser Leu Asn Gly Thr Ser Met Ala Thr Pro His Val Ala Gly Ala
       210                 215                 220
   Ala Ala Leu Val Lys Gln Lys Asn Pro Ser Trp Ser Asn Val Gln Ile
   225                 230                 235                 240
   Arg Asn His Leu Lys Asn Thr Ala Thr Ser Leu Gly Ser Thr Asn Leu
                   245                 250                 255
   Tyr Gly Ser Gly Leu Val Asn Ala Glu Ala Ala Thr Arg
               260                 265

## Beschreibung

Die Erfindung liegt auf dem Gebiet der festen Textilwaschmittel. Die Erfindung betrifft insbesondere feste enzymhaltige Textilwaschmittel, die bestimmte Proteasen enthalten, und schlägt ferner Verfahren vor, in denen solche Mittel angewendet werden. Die Erfindung betrifft weiterhin die Verwendung der bestimmten Proteasen in festen Textilwaschmitteln.

Für Wasch- und Reinigungsmittel werden bevorzugt Proteasen vom Subtilisin-Typ eingesetzt. Die in den aus dem Stand der Technik bekannten Wasch- oder Reinigungsmitteln eingesetzten Proteasen stammen entweder ursprünglich aus Mikroorganismen, beispielsweise der Gattungen Bacillus, Streptomyces, Humicola oder Pseudomonas, und/oder werden nach an sich bekannten biotechnologischen Verfahren durch geeignete Mikroorganismen produziert, beispielsweise durch transgene Expressionswirte der Gattungen Bacillus oder durch filamentöse Pilze.

In der internationalen Patentanmeldung WO 95/23221 sind Proteasen bzw. Protease-Varianten vom Subtilisin-Typ aus Bacillus lentus DSM 5483 offenbart, die für den Einsatz in Wasch- oder Reinigungsmitteln geeignet sind. Unter diesen Proteasen ist auch eine, die einen Aminosäureaustausch R99E, A, S oder G aufweisen kann. Die Waschmittel können fest oder flüssig sein. Jedoch geht aus dieser Schrift nicht unmittelbar und eindeutig ein festes Textilwaschmittel hervor, welches eine Protease, die an Position 99 die Aminosäure Glutaminsäure (E) oder Asparaginsäure (D) oder die Aminosäure Asparagin (N) oder Glutamin (Q) oder die Aminosäure Alanin (A) oder Glycin (G) oder Serin (S) enthält, und einen bestimmten pH-Wert aufweist.

Die genannten Proteasen sind als Inhaltsstoff von flüssigen Wasch- oder Reinigungsmitteln bekannt. Beispielsweise werden vorteilhafte flüssige Wasch- oder Reinigungsmittel, welche eine der genannten Proteasen in Kombination mit Cellulasen enthalten, in der internationalen Patentanmeldung WO 2012/080201 beschrieben. Flüssige Wasch- oder Reinigungsmittel, welche eine der genannten Proteasen in Kombination mit Amylasen aufweist, werden in WO 2012/080202 beschrieben.

Vorteile der genannten Proteasen gegenüber herkömmlichen Proteasen des Standes der Technik in festen automatischen Geschirrspülmitteln werden in der deutschen Patentanmeldung DE 102012215107.9 offenbart. Feste automatische Geschirrspülmittel weisen in der Regel hohe pH-Werte von 10 oder mehr auf.
Erstaunlicherweise konnte bis heute kein Vorteil der genannten Proteasen in festen Textilwaschmitteln mit ähnlichem pH-Profil wie in automatischen festen Geschirrspülmitteln gefunden werden. Hohe pH-Werte in der Waschflotte und somit auch hohe Alkaliwerte in den Waschmitteln tragen bekannterweise zur Waschleistung bei. Im Hinblick auf die Textilpflege können sie allerdings negative Auswirkungen haben, so dass im Hinblick auf Textilpflege und Textilschonung niedrigere pH-Werte wünschenswert sind.
Es bestand daher die Aufgabe, die Waschleistung von festen Textilwaschmitteln, welche einen geringeren pH-Wert in 1 Gew.-%iger Lösung in entionisiertem Wasser bei 20 °C aufweisen, zu verbessern.
Überraschenderweise wurde festgestellt, dass die oben genannten Proteasen in festen Textilwaschmitteln mit niedrigerem pH-Wert in 1 Gew.-%iger Lösung in entionisiertem Wasser bei 20 °C Vorteile gegenüber den bisherigen Proteasen, welche bisher in festen Textilwaschmitteln eingesetzt werden, zeigen.
Ein erster Gegenstand der Erfindung ist daher ein festes Textilwaschmittel, enthaltend
(a1) eine Protease, die eine Aminosäuresequenz umfasst, die zu der in SEQ ID NO. 1 angegebenen Aminosäuresequenz zu mindestens 80% identisch ist und die an Position 99 in der Zählung gemäß SEQ ID NO. 1 die Aminosäure Glutaminsäure (E) oder Asparaginsäure (D) aufweist, oder (a2) eine Protease, die eine Aminosäuresequenz umfasst, die zu der in SEQ ID NO. 1 angegebenen Aminosäuresequenz zu mindestens 80% identisch ist und die an Position 99 in der Zählung gemäß SEQ ID NO. 1 die Aminosäure Asparagin (N) oder Glutamin (Q) aufweist, wobei das feste Textilwaschmittel in 1 Gew.-%iger Lösung in entionisiertem Wasser bei 20 °C einen pH-Wert in einem Bereich von 4,0 bis unter 10 aufweist.
Erfindungsgemäße feste Textilwaschmittel zeigen insbesondere auch dann Leistungsvorteile gegenüber anderen festen Textilwaschmitteln, wenn die festen Textilwaschmittel mindestens ein zusätzliches Enzym derselben oder einer anderen Art, also beispielsweise Amylase, Cellulase, Lipase, Mannanase oder Pektinase enthalten, wobei die Auflistung der weiteren Enzyme nicht vollständig ist. Daher ist es bevorzugt, dass die erfindungsgemäßen festen Textilwaschmittel mindestens ein zusätzliches Enzym derselben Art (also eine weitere Protease) oder einer anderen Art enthalten.
Die erfindungsgemäßen Mittel weisen insbesondere auch dann einen Vorteil gegenüber herkömmlichen festen Textilwaschmitteln auf, wenn sie keine Bleiche und insbesondere keine anorganische oder organische Peroxidbleichequelle enthalten.

In einer vorteilhaften Ausführungsform der Erfindung weisen die festen Textilwaschmittel einen pH-Wert (1 Gew.-%ige Lösung in entionisiertem Wasser bei 20 °C) von 6,5 bis 9,5 und insbesondere von 7,0 bis 9,0 auf.
Ein erfindungsgemäßes Mittel weist insbesondere vorteilhafte Reinigungsleistungen an Protease-sensitiven Anschmutzungen auf. Eine solche Reinigungsleistung hinsichtlich mindestens einer Protease-sensitiven Anschmutzung tritt insbesondere auch bei niedrigen Temperaturen, beispielsweise bei Waschtemperaturen von 10°C bis 50°C, bevorzugt von 10°C bis 40°C oder von 20°C bis 40°C auf. Ein solches Mittel ermöglicht daher eine zufriedenstellende oder verbesserte Entfernung von mindestens einer, bevorzugt von mehreren Protease-sensitiven Anschmutzungen auf Textilien. Unter Reinigungsleistung wird im Rahmen der Erfindung die Aufhellungsleistung an einer oder mehreren Anschmutzungen, insbesondere Wäscheanschmutzungen, verstanden. Beispiele für solche Anschmutzungen sind Blut auf Baumwolle oder Schokolade-Milch/Ruß auf Baumwolle, Kakao auf Baumwolle oder Porridge auf Baumwolle.
Die in einem erfindungsgemäßen festen Textilwaschmittel enthaltene Protease umfasst eine Aminosäuresequenz, die zu der in SEQ ID NO. 1 angegebenen Aminosäuresequenz zu mindestens 80% identisch ist und die an Position 99 in der Zählung gemäß SEQ ID NO. 1 die Aminosäure Glutaminsäure (E) oder Asparaginsäure (D) oder die Aminosäure Asparagin (N) oder Glutamin (Q) aufweist. Zunehmend bevorzugt ist die Aminosäuresequenz zu mindestens 81%, 82%, 83%, 84%, 85%, 86%, 87%, 88%, 89%, 90%, 91%, 92%, 93%, 94%, 95%, 96%, 97%, 98% und ganz besonders bevorzugt zu 99% identisch zu der in SEQ ID NO. 1 angegebenen Aminosäuresequenz. SEQ ID NO. 1 ist die Sequenz der reifen (maturen) alkalischen Protease aus Bacillus lentus DSM 5483, die in der internationalen Patentanmeldung WO 92/21760 offenbart ist und auf deren Offenbarung hiermit ausdrücklich Bezug genommen wird.
Eine in einem erfindungsgemäßen festen Textilwaschmittel enthaltene Protease weist eine proteolytische Aktivität auf, das heißt, sie ist zur Hydrolyse von Peptidbindungen eines Polypeptids bzw. Proteins befähigt. Sie ist daher ein Enzym, welches die Hydrolyse von Peptidbindungen katalysiert und dadurch in der Lage ist, Peptide oder Proteine zu spalten. Sie ist insbesondere eine Subtilase und besonders bevorzugt ein Subtilisin.
In einer weiteren Ausführungsform der Erfindung ist das feste Textilwaschmittel dadurch gekennzeichnet, dass die Protease ferner in der Zählung gemäß SEQ ID NO. 1 mindestens eine der folgenden Aminosäuren aufweist:
(a) Threonin an Position 3 (3T),
(b) Isoleucin an Position 4 (4I),
(c) Alanin, Threonin oder Arginin an Position 61 (61A, 61T oder 61R),
(d) Asparaginsäure oder Glutaminsäure an Position 154 (154D oder 154E),
(e) Prolin an Position 188 (188P),
(f) Methionin an Position 193 (193M),
(g) Isoleucin an Position 199 (199I),
(h) Asparaginsäure, Glutaminsäure oder Glycin an Position 211 (211D, 211E oder 211G),
(i) Kombinationen der Aminosäuren (a) bis (h).

Neben einer der genannten Aminosäuren an Position 99 weist die Protease daher eine oder mehrere der vorstehend genannten Aminosäuren an den jeweiligen Positionen auf. Diese Aminosäuren können weitere vorteilhafte Eigenschaften bewirken und/oder bereits vorhandene Eigenschaften noch verstärken. Insbesondere bewirken die vorstehend genannten Aminosäuren eine Steigerung der proteolytischen Aktivität und/oder der Stabilität der Protease in einem festen Textilwaschmittel bzw. in der durch dieses Waschmittel gebildeten Waschflotte. Durch den Zusatz einer solchen Protease zu einem festen Textilwaschmittel wird somit ebenfalls ein besonders lagerstabiles festes Textilwaschmittel erhalten.

Die Aminosäurepositionen werden durch ein Alignment der Aminosäuresequenz der einzusetzenden Protease mit der Aminosäuresequenz der Protease aus Bacillus lentus, wie sie in SEQ ID NO. 1 angegeben ist, definiert. Da die Protease aus Bacillus lentus im Stand der Technik ein wichtiges Referenzmolekül zur Beschreibung von Proteasen und von Aminosäureveränderungen darstellt ist es vorteilhaft, in der Zuordnung der Aminosäurepositionen auf die Zählung der Protease aus Bacillus lentus (SEQ ID NO. 1) Bezug zu nehmen. Weiterhin richtet sich die Zählung nach dem reifen (maturen) Protein. Diese Zuordnung ist insbesondere auch anzuwenden, wenn die Aminosäuresequenz der einzusetzenden Protease eine höhere Zahl von Aminosäurenresten umfasst als die Protease aus Bacillus lentus gemäß SEQ ID NO. 1. Ausgehend von den genannten Positionen in der Aminosäuresequenz der Protease aus Bacillus lentus sind die Aminosäurepositionen in einer erfindungsgemäß einzusetzenden Protease diejenigen, die eben diesen Positionen in einem Alignment zugeordnet sind.

Neben Position 99 besonders vorteilhafte Positionen sind demnach die Positionen 3, 4, 61, 154, 188, 193, 199 und 211, zuzuordnen in einem Alignment mit SEQ ID NO. 1 und damit in der Zählung gemäß SEQ ID NO. 1. In den genannten Positionen liegen in dem Wildtypmolekül der Protease aus Bacillus lentus folgende Aminosäurereste: S3, V4, G61, S154, A188, V193, V199, und L211. Besonders bevorzugt sind die Aminosäuren 3T, 4I, 61A, 154D, 154E, 211D, 211G und 211E, sofern die entsprechenden Positionen in einer erfindungsgemäß einzusetzenden Protease nicht schon von einer dieser bevorzugten Aminosäuren eingenommen werden. Die Austausche 3T und 4I führen beispielsweise über einen Stabilisierungseffekt auf das Molekül zu einer Verbesserung der Lagerstabilität und der Reinigungsleistung der Protease und damit zu einer verbesserten Reinigungsleistung eines festen Textilwaschmittels, welches die Protease enthält.

Sind eine oder mehrere der vorstehend genannten Aminosäuren an der jeweiligen Position verwirklicht, ergeben sich zusätzlich zu Position 99 weitere Sequenzabweichungen von SEQ ID NO. 1, da SEQ ID NO. 1 eine andere Aminosäure in der jeweiligen Position aufweist. In Abhängigkeit von der Anzahl der vorliegenden Sequenzabweichungen von SEQ ID NO. 1 ergeben sich daher unterschiedliche maximale Identitätswerte, die eine erfindungsgemäß einzusetzende Protease zu SEQ ID NO. 1 aufweisen kann, selbst wenn sie in allen übrigen Aminosäuren mit SEQ ID NO. 1 übereinstimmen sollte. Dieser Sachverhalt ist für jede mögliche Kombination der vorgeschlagenen Aminosäuren im Einzelfall zu berücksichtigen und ist ferner auch abhängig von der Länge der Aminosäuresequenz der Protease. Beispielsweise beträgt die maximale Identität bei einer, zwei, drei, vier, fünf, sechs, sieben, acht oder neun Sequenzveränderungen 99,63%, 99,26%, 98,88%, 98,51%, 98,14%, 97,77%, 97,40%, 97,03% oder 96,65% bei einer 269 Aminosäuren langen Aminosäuresequenz, beziehungsweise 99,64%, 99,27%, 98,91%, 98,55%, 98,18%, 97,82%, 97,45%, 97,09% oder 96,73% bei einer 275 Aminosäuren langen Aminosäuresequenz.

Die Bestimmung der Identität von Nukleinsäure- oder Aminosäuresequenzen erfolgt durch einen Sequenzvergleich. Solch ein Vergleich erfolgt dadurch, dass ähnliche Abfolgen in den Nukleotidsequenzen oder Aminosäuresequenzen einander zugeordnet werden. Dieser Sequenzvergleich erfolgt vorzugsweise basierend auf dem im Stand der Technik etablierten und üblicherweise genutzten BLAST-Algorithmus (vgl. beispielsweise Altschul, S.F., Gish, W., Miller, W., Myers, E.W. & Lipman, D.J. (1990) "Basic local alignment search tool." J. Mol. Biol. 215:403-410, und Altschul, Stephan F.,Thomas L. Madden, Alejandro A. Schaffer, Jinghui Zhang, Hheng Zhang, Webb Miller, and David J. Lipman (1997): "Gapped BLAST and PSI-BLAST: a new generation of protein database search programs"; Nucleic Acids Res., 25, S.3389-3402) und geschieht prinzipiell dadurch, dass ähnliche Abfolgen von Nukleotiden oder Aminosäuren in den Nukleinsäure- bzw. Aminosäuresequenzen einander zugeordnet werden. Eine tabellarische Zuordnung der betreffenden Positionen wird als Alignment bezeichnet. Ein weiterer im Stand der Technik verfügbarer Algorithmus ist der FASTA-Algorithmus. Sequenzvergleiche (Alignments), insbesondere multiple Sequenzvergleiche, werden üblicherweise mit Computerprogrammen erstellt. Häufig genutzt werden beispielsweise die Clustal-Serie (vgl. beispielsweise Chenna et al. (2003): Multiple sequence alignment with the Clustal series of programs. Nucleic Acid Research 31, 3497-3500), T-Coffee (vgl. beispielsweise Notredame et al. (2000): T-Coffee: A novel method for multiple sequence alignments. J. Mol. Biol. 302, 205-217) oder Programme, die auf diesen Programmen bzw. Algorithmen basieren. Im Rahmen der vorliegenden Erfindung werden Sequenzvergleiche und Alignments bevorzugt mit dem Computer-Programm Vector NTI® Suite 10.3 (Invitrogen Corporation, 1600 Faraday Avenue, Carlsbad, Kalifornien, USA) mit den vorgegebenen Standard (Default)-Parametern erstellt.

Solch ein Vergleich erlaubt eine Aussage über die Ähnlichkeit der verglichenen Sequenzen zueinander. Sie wird üblicherweise in Prozent Identität, das heißt dem Anteil der identischen Nukleotide oder Aminosäurereste an denselben bzw. in einem Alignment einander entsprechenden Positionen, angegeben. Der weiter gefasste Begriff der Homologie bezieht bei Aminosäuresequenzen konservierte Aminosäure-Austausche in die Betrachtung mit ein, also Aminosäuren mit ähnlichen Eigenschaften, da diese innerhalb des Proteins meist ähnliche Aktivitäten bzw. Funktionen ausüben. Daher kann die Ähnlichkeit der verglichenen Sequenzen auch Prozent Homologie oder Prozent Ähnlichkeit angegeben sein. Identitäts- und/oder Homologieangaben können über ganze Polypeptide oder Gene oder nur über einzelne Bereiche getroffen werden. Homologe bzw. identische Bereiche von verschiedenen Nukleinsäure- oder Aminosäuresequenzen sind daher durch Übereinstimmungen in den Sequenzen definiert. Sie weisen oftmals gleiche oder ähnliche Funktionen auf. Sie können klein sein und nur wenige Nukleotide bzw. Aminosäuren umfassen. Oftmals üben solche kleinen Bereiche für die Gesamtaktivität des Proteins essentielle Funktionen aus. Es kann daher sinnvoll sein, Sequenzübereinstimmungen nur auf einzelne, gegebenenfalls kleine Bereiche zu beziehen. Soweit nicht anders angegeben beziehen sich Identitäts- bzw. Homologieangaben in der vorliegenden Anmeldung aber auf die Gesamtlänge der jeweils angegebenen Nukleinsäureoder Aminosäuresäuresequenz.

In einer weiteren Ausführungsform der Offenbarung ist das feste Textilwaschmittel dadurch gekennzeichnet, dass die Protease eine Aminosäuresequenz umfasst, die zu der in SEQ ID NO. 1 angegebenen Aminosäuresequenz wie vorstehend angegeben identisch ist und die aus einer Protease gemäß SEQ ID NO. 1 durch ein- oder mehrfache konservative Aminosäuresubstitution erhalten wird bzw. erhältlich ist, wobei die Protease an Position 99 noch immer eine der für diese Position vorgesehenen Aminosäuren aufweist wie vorstehend beschrieben. Der Begriff "konservative Aminosäuresubstitution" bedeutet den Austausch (Substitution) eines Aminosäurerestes gegen einen anderen Aminosäurerest, wobei dieser Austausch nicht zu einer Änderung der Polarität oder Ladung an der Position der ausgetauschten Aminosäure führt, z. B. der Austausch eines unpolaren Aminosäurerestes gegen einen anderen unpolaren Aminosäurerest. Konservative Aminosäuresubstitutionen im Rahmen der Offenbarung umfassen beispielsweise: G=A=S, I=V=L=M, D=E, N=Q, K=R, Y=F, S=T, G=A=I=V=L=M=Y=F=W=P=S=T.
Zahlreiche Proteasen und insbesondere Subtilisine werden als sogenannte Präproteine, also zusammen mit einem Propeptid und einem Signalpeptid gebildet, wobei die Funktion des Signalpeptids üblicherweise darin besteht, die Ausschleusung der Protease aus der sie produzierenden Zelle in das Periplasma oder das die Zelle umgebende Medium zu gewährleisten, und das Propeptid üblicherweise für die korrekte Faltung der Protease nötig ist. Das Signalpeptid und das Propeptid sind in der Regel der N-terminale Teil des Präproteins. Das Signalpeptid wird unter natürlichen Bedigungen durch eine Signalpeptidase von der übrigen Protease abgespalten. Anschließend erfolgt die durch das Propeptid unterstützte korrekte endgültige Faltung der Protease. Die Protease ist dann in ihrer aktiven Form und spaltet das Propeptid selbst ab. Nach der Abspaltung des Propeptids übt die dann reife (mature) Protease, insbesondere Subtilisin, ihre katalytische Aktivität ohne die ursprünglich vorhandenen N-terminalen Aminosäuren aus. Für technische Anwendungen allgemein und insbesondere im Rahmen der Erfindung sind die reifen (maturen) Proteasen, d.h. die nach ihrer Herstellung prozessierten Enzyme, gegenüber den Präproteinen bevorzugt. Die Proteasen können ferner von den sie produzierenden Zellen nach der Herstellung der Polypeptidkette modifiziert werden, beispielsweise durch Anknüpfung von Zuckermolekülen, Formylierungen, Aminierungen, usw. Solche Modifikationen sind posttranslationale Modifikationen und können, müssen jedoch nicht einen Einfluss auf die Funktion der Protease ausüben.

Ein erfindungsgemäßes Mittel enthält die Protease zunehmend bevorzugt in einer Menge von 1 x 10⁻⁸ - 5 Gew.-%, von 0,0001-1 Gew.-%, von 0,0005-0,5 Gew.-%, von 0,001 bis 0,1 Gew.-%, jeweils bezogen auf aktives Protein. Die Proteinkonzentration kann mit Hilfe bekannter Methoden, zum Beispiel dem BCA-Verfahren (Bicinchoninsäure; 2,2'-Bichinolyl-4,4'-dicarbonsäure) oder dem Biuret-Verfahren (A. G. Gornall, C. S. Bardawill und M.M. David, J. Biol. Chem., 177 (1948), S. 751-766) bestimmt werden. Die Bestimmung der Aktivproteinkonzentration erfolgte diesbezüglich über eine Titration der aktiven Zentren unter Verwendung eines geeigneten irreversiblen Inhibitors (für Proteasen beispielsweise Phenylmethylsulfonylfluorid (PMSF)) und Bestimmung der Restaktivität (vgl. M. Bender et al., J. Am. Chem. Soc. 88, 24 (1966), S. 5890-5913).

Die Protease und/oder das mindestens eine zusätzliche Enzym gleicher oder anderer Art kann an Trägerstoffen adsorbiert und/oder in Hüllsubstanzen eingebettet sein, um sie gegen vorzeitige Inaktivierung zu schützen. In der Waschflotte, also unter Anwendungsbedingungen, wird das Enzym dann freigesetzt und kann seine katalytische Wirkung entfalten. Die so konfektionierten Protease der eingangs genannten Art kann in Mengen von 0,05 bis 5 Gew.-%, vorzugsweise in Mengen von 0,05 bis 2 Gew.-% in dem festen Textilwaschmittel enthalten sein. Bevorzugte Konfektionierungsformen weisen 0,05 bis 15 Gew.-% und insbesondere bis 10 Gew.-% aktives Protein der genannten Protease auf.

Zusätzlich zu dieser Protease können die festen Textilwaschmittel weitere Enzyme enthalten und weisen bevorzugte feste Textilwaschmittel mindestens ein weiteres Enzym auf, wobei es sich bei dem mindestens einen weiteren Enzym um eine weitere Protease, um eine Amylase, Cellulase, Hemicellulase, Mannanase, Tannase, Xylanase, Xanthanase, Xyloglucanase, ß-Glucosidase, Pektinase, Carrageenase, Perhydrolase, Oxidase, Oxidoreduktase oder eine Lipase sowie deren Gemische handelt. Weitere Enzyme sind in dem Mittel vorteilhafterweise jeweils in einer Menge von 1 x 10⁻⁸ bis 5 Gewichts-Prozent bezogen auf aktives Protein enthalten. Zunehmend bevorzugt ist jedes weitere Enzym in einer Menge von 1 x 10⁻⁷-3 Gew.-%, von 0,00001-1 Gew.-%, von 0,00005-0,5 Gew.-%, von 0,0001 bis 0,1 Gew.-% und besonders bevorzugt von 0,0001 bis 0,05 Gew.-% , bezogen auf aktives Protein, in den festen Textilwaschmitteln enthalten. Besonders bevorzugt zeigen die Enzyme synergistische Reinigungsleistungen gegenüber bestimmten Anschmutzungen oder Flecken, d.h. die in der Mittelzusammensetzung enthaltenen Enzyme unterstützen sich in ihrer Reinigungsleistung gegenseitig. Ganz besonders bevorzugt liegt ein solcher Synergismus zwischen der erfindungsgemäß enthaltenen Protease und einem weiteren Enzym, darunter insbesondere zwischen der genannten Protease und einer Amylase und/oder einer Lipase und/oder einer Mannanase und/oder einer Cellulase und/oder einer Pektinase, vor. Synergistische Effekte können nicht nur zwischen verschiedenen Enzymen, sondern auch zwischen einem oder mehreren Enzymen und weiteren Inhaltsstoffen des erfindungsgemäßen Mittels auftreten. Bevorzugte feste Textilwaschmittel weisen daher mindestens eine Protease und mindestens eine Amylase auf. In einer weiteren bevorzugten Ausführungsform der Erfindung weisen feste Textilwaschmittel mindestens eine Protease und mindestens eine Cellulase auf. In einer weiteren bevorzugten Ausführungsform weisen feste Textilwaschmittel mindestens eine Protease und mindestens eine Lipase auf. Besonders bevorzugt sind feste Textilwaschmittel, welche 3 bis 10 verschiedene Enzyme aufweisen, wobei feste Textilwaschmittel, welche 3 bis 10 unterschiedliche Enzymarten aufweisen, im Hinblick auf die Reinigungsleistung gegenüber einem sehr großen Fleckenspektrum von besonderer Bevorzugung sein können.

Die festen Textilwaschmittel können als pulverförmige bis granulare Feststoffe, aber auch in verdichteter oder in nachverdichteter Form vorliegen. Im Prinzip können sie alle bekannten und in derartigen Mitteln üblichen Inhaltsstoffe enthalten. Die erfindungsgemäßen festen Textilwaschmittel können insbesondere Buildersubstanzen, oberflächenaktive Tenside, die oben bereits erwähnten Enzyme, Enzymstabilisatoren, Sequestrierungsmittel, Elektrolyte, pH-Regulatoren und weitere Hilfsstoffe, wie optische Aufheller, Vergrauungsinhibitoren, Farbübertragungsinhibitoren, Schaumregulatoren, sowie Farb- und Duftstoffe enthalten. Auch wenn Bleiche-freie Mittel und insbesondere Peroxid-freie Mittel besonders bevorzugt sind, so werden Bleichmittel wie PersauerstoffVerbindungen und Persauerstoff-Aktivatoren, aber auch metallhaltige oder metallfreie Bleichkatalysatoren nicht generell ausgeschlossen.

Als geeignete organische oder anorganische Persauerstoffverbindungen kommen organische Persäuren beziehungsweise persaure Salze organischer Säuren, wie Phthalimidopercapronsäure, Perbenzoesäure oder Salze der Diperdodecandisäure, insbesondere abe anorganische Persauerstoffverbindungen wie Wasserstoffperoxid und unter den Reinigungsbedingungen Wasserstoffperoxid abgebende anorganische Salze, wie Perborat, Percarbonat und/oder Persilikat, und Wasserstoffperoxid-Einschlußverbindungen, wie H₂O₂-Harnstoffaddukt in Betracht. Wasserstoffperoxid kann dabei auch mit Hilfe eines enzymatischen Systems, das heißt einer Oxidase und ihres Substrats, erzeugt werden. Sofern feste Persauerstoffverbindungen eingesetzt werden sollen, können diese in Form von Pulvern oder Granulaten verwendet werden, die auch in im Prinzip bekannter Weise umhüllt sein können. Die Persauerstoffverbindungen können als solche oder in Form diese enthaltender Mittel, die prinzipiell alle üblichen Wasch-, Reinigungs- oder Desinfektionsmittelbestandteile enthalten können, zu der Wasch- beziehungsweise Reinigungslauge zugegeben werden. Besonders bevorzugt wird Alkalipercarbonat oder Alkaliperborat-Monohydrat eingesetzt. Falls ein erfindungsgemäßes festes Textilwaschmittel Persauerstoffverbindungen enthält, sind diese vorzugsweise in Mengen von 0,1 Gew.-% bis 20 Gew.-% vorhanden.

Geeignete Bleichaktivatoren sind Verbindungen, die O- und/oder N-Acylgruppen und/oder gegebenenfalls substituierte Benzoylgruppen tragen. Bevorzugt sind mehrfach acylierte Alkylendiamine, insbesondere Tetraacetylethylendiamin (TAED), acylierte Glykolurile, insbesondere Tetraacetylglykoluril (TAGU), acylierte Triazinderivate, insbesondere 1,5-Diacetyl-2,4-dioxohexahydro-1,3,5-triazin (DADHT), N-Acylimide, insbesondere N-Nonanoylsuccinimid (NOSI), acylierte Phenolsulfonate oder -carboxylate beziehungsweise die Sulfon- oder Carbonsäuren von diesen, insbesondere Nonanoyl- oder Isononanoyl- oder Lauroyloxybenzolsulfonat (NOBS beziehungsweise iso-NOBS beziehungsweise LOBS), 4-(2-Decanoyloxyethoxycarbonyloxy)-benzolsulfonat (DECOBS) oder Decanoyloxybenzoat (DOBA), acylierte mehrwertige Alkohole, insbesondere Triacetin, Ethylenglykoldiacetat und 2,5-Diacetoxy-2,5-dihydrofuran sowie acetyliertes Sorbitol und Mannitol und deren Mischungen (SORMAN), acylierte Zuckerderivate, insbesondere Pentaacetylglukose (PAG), Pentaacetylfruktose, Tetraacetylxylose und Octaacetyllactose, acetyliertes, gegebenenfalls N-alkyliertes Glucamin und Gluconolacton, N-acylierte Lactame, beispielsweise N-Benzoylcaprolactam, Nitrile, aus denen sich Perimidsäuren bilden, insbesondere Aminoacetonitrilderivate mit quaterniertem Stickstoffatom, und/oder sauerstoffübertragende Sulfonimine und/oder Acylhydrazone. Diese Bleichaktivatoren können in den Mitteln vorzugsweise bis zu 10 Gew.-% enthalten sein.

Die erfindungsgemäßen Mittel können ein oder mehrere Tenside enthalten, wobei insbesondere anionische Tenside, nichtionische Tenside und deren Gemische in Frage kommen. Geeignete nichtionische Tenside sind insbesondere Alkylglykoside und Ethoxylierungs- und/oder Propoxylierungsprodukte von Alkylglykosiden oder linearen oder verzweigten Alkoholen mit jeweils 8 bis etwa 18 C-Atomen im Alkylteil und 3 bis 20, vorzugsweise 4 bis 10 Alkylethergruppen. Weiterhin sind entsprechende Ethoxylierungs- und/oder Propoxylierungsprodukte von N-Alkyl-aminen, vicinalen Diolen, Fettsäureestern und Fettsäureamiden, die hinsichtlich des Alkylteils den genannten langkettigen Alkoholderivaten entsprechen, sowie von Alkylphenolen mit 5 bis 12 C-Atomen im Alkylrest brauchbar.

Geeignete anionische Tenside sind insbesondere Seifen und solche, die Sulfat- oder SulfonatGruppen mit bevorzugt Alkaliionen als Kationen enthalten. Verwendbare Seifen sind bevorzugt die Alkalisalze der gesättigten oder ungesättigten Fettsäuren mit 12 bis 18 C-Atomen. Derartige Fettsäuren können auch in nicht vollständig neutralisierter Form eingesetzt werden. Zu den brauchbaren Tensiden des Sulfat-Typs gehören die Salze der Schwefelsäurehalbester von Fettalkoholen mit 12 bis 18 C-Atomen und die Sulfatierungsprodukte der genannten nichtionischen Tenside mit niedrigem Ethoxylierungsgrad. Zu den verwendbaren Tensiden vom Sulfonat-Typ gehören lineare Alkylbenzolsulfonate mit 9 bis 14 C-Atomen im Alkylteil, Alkansulfonate mit 12 bis 18 C-Atomen, sowie Olefinsulfonate mit 12 bis 18 C-Atomen, die bei der Umsetzung entsprechender Monoolefine mit Schwefeltrioxid entstehen, sowie alpha-Sulfofettsäureester, die bei der Sulfonierung von Fettsäuremethyl- oder -ethylestern entstehen.

Derartige Tenside sind in den erfindungsgemäßen festen Textilwaschmitteln in Mengenanteilen von vorzugsweise 5 Gew.-% bis 50 Gew.-% und insbesondere von 8 Gew.-% bis 30 Gew.-% enthalten.

Ein erfindungsgemäßes Mittel enthält vorzugsweise mindestens einen wasserlöslichen und/oder wasserunlöslichen, organischen und/oder anorganischen Builder. Zu den wasserlöslichen organischen Buildersubstanzen gehören Polycarbonsäuren, insbesondere Citronensäure, Zuckersäuren und Carboxymethylinuline, monomere und polymere Aminopolycarbonsäuren, insbesondere Glycindiessigsäure, Methylglycindiessigsäure, Nitrilotriessigsäure, Iminodisuccinate wie Ethylendiamin-N,N'-dibernsteinsäure und Hydroxyiminodisuccinate, Ethylendiamintetraessigsäure sowie Polyasparaginsäure, Polyphosphonsäuren, insbesondere Aminotris(methylenphosphonsäure), Ethylendiamintetrakis(methylenphosphonsäure), Lysintetra(methylenphosphosäure) und 1-Hydroxyethan-1,1-diphosphonsäure, polymere Hydroxyverbindungen wie Dextrin sowie polymere (Poly-)carbonsäuren, insbesondere durch Oxidation von Polysacchariden zugängliche Polycarboxylate, polymere Acrylsäuren, Methacrylsäuren, Maleinsäuren und Mischpolymere aus diesen, die auch geringe Anteile polymerisierbarer Substanzen ohne Carbonsäurefunktionalität einpolymerisiert enthalten können. Die relative mittlere Molekülmasse (hier und im Folgenden: Gewichtsmittel) der Homopolymeren ungesättiger Carbonsäuren liegt im allgemeinen zwischen 5 000 g/mol und 200 000 g/mol, die der Copolymeren zwischen 2 000 g/mol und 200 000 g/mol, vorzugsweise 50 000 g/mol bis 120 000 g/mol, jeweils bezogen auf freie Säure. Ein besonders bevorzugtes Acrylsäure-Maleinsäure-Copolymer weist eine relative mittlere Molekülmasse von 50 000 g/mol bis 100 000 g/mol auf. Geeignete, wenn auch weniger bevorzugte Verbindungen dieser Klasse sind Copolymere der Acrylsäure oder Methacrylsäure mit Vinylethern, wie Vinylmethylethern, Vinylester, Ethylen, Propylen und Styrol, in denen der Anteil der Säure mindestens 50 Gew.-% beträgt. Als wasserlösliche organische Buildersubstanzen können auch Terpolymere eingesetzt werden, die als Monomere zwei ungesättigte Säuren und/oder deren Salze sowie als drittes Monomer Vinylalkohol und/ oder ein Vinylalkohol-Derivat oder ein Kohlenhydrat enthalten. Das erste saure Monomer beziehungsweise dessen Salz leitet sich von einer monoethylenisch ungesättigten C₃-C₈-Carbonsäure und vorzugsweise von einer C₃-C₄-Monocarbonsäure, insbesondere von (Meth)-acrylsäure ab. Das zweite saure Monomer beziehungsweise dessen Salz kann ein Derivat einer C₄-C₈-Dicarbonsäure sein, wobei Maleinsäure besonders bevorzugt ist. Die dritte monomere Einheit wird in diesem Fall von Vinylalkohol und/oder vorzugsweise einem veresterten Vinylalkohol gebildet. Insbesondere sind Vinylalkohol-Derivate bevorzugt, welche einen Ester aus kurzkettigen Carbonsäuren, beispielsweise von C₁-C₄-Carbonsäuren, mit Vinylalkohol darstellen. Bevorzugte Polymere enthalten dabei 60 Gew.-% bis 95 Gew.-%, insbesondere 70 Gew.-% bis 90 Gew.-% (Meth)acrylsäure bzw.

(Meth)acrylat, besonders bevorzugt Acrylsäure bzw. Acrylat, und Maleinsäure bzw. Maleinat sowie 5 Gew.-% bis 40 Gew.-%, vorzugsweise 10 Gew.-% bis 30 Gew.-% Vinylalkohol und/oder Vinylacetat. Ganz besonders bevorzugt sind dabei Polymere, in denen das Gewichtsverhältnis von (Meth)acrylsäure beziehungsweise (Meth)acrylat zu Maleinsäure beziehungsweise Maleinat zwischen 1:1 und 4:1, vorzugsweise zwischen 2:1 und 3:1 und insbesondere 2:1 und 2,5:1 liegt. Dabei sind sowohl die Mengen als auch die Gewichtsverhältnisse auf die Säuren bezogen. Das zweite saure Monomer beziehungsweise dessen Salz kann auch ein Derivat einer Allylsulfonsäure sein, die in 2-Stellung mit einem Alkylrest, vorzugsweise mit einem C₁-C₄-Alkylrest, oder einem aromatischen Rest, der sich vorzugsweise von Benzol oder Benzol-Derivaten ableitet, substituiert ist. Bevorzugte Terpolymere enthalten dabei 40 Gew.-% bis 60 Gew.-%, insbesondere 45 bis 55 Gew.- % (Meth)acrylsäure beziehungsweise (Meth)acrylat, besonders bevorzugt Acrylsäure beziehungsweise Acrylat, 10 Gew.-% bis 30 Gew.-%, vorzugsweise 15 Gew.-% bis 25 Gew.-% Methallylsulfonsäure bzw. Methallylsulfonat und als drittes Monomer 15 Gew.-% bis 40 Gew.-%, vorzugsweise 20 Gew.-% bis 40 Gew.-% eines Kohlenhydrats. Dieses Kohlenhydrat kann dabei beispielsweise ein Mono-, Di-, Oligo- oder Polysaccharid sein, wobei Mono-, Di- oder Oligosaccharide bevorzugt sind. Besonders bevorzugt ist Saccharose. Durch den Einsatz des dritten Monomers werden vermutlich Sollbruchstellen in das Polymer eingebaut, die für die gute biologische Abbaubarkeit des Polymers verantwortlich sind. Diese Terpolymere weisen im Allgemeinen eine relative mittlere Molekülmasse zwischen 1 000 g/mol und 200 000 g/mol, vorzugsweise zwischen 200 g/mol und 50 000 g/mol auf. Weitere bevorzugte Copolymere sind solche, die als Monomere Acrolein und Acrylsäure/Acrylsäuresalze beziehungsweise Vinylacetat aufweisen. Alle genannten Säuren werden in der Regel in Form ihrer wasserlöslichen Salze, insbesondere ihre Alkalisalze, eingesetzt.

Derartige organische Buildersubstanzen können gewünschtenfalls in Mengen bis zu 40 Gew.-%, insbesondere bis zu 25 Gew.-% und vorzugsweise von 1 Gew.-% bis 8 Gew.-% enthalten sein.

Als wasserunlösliche anorganische Buildermaterialien werden insbesondere kristalline oder amorphe, wasserdispergierbare Alkalialumosilikate, vorzugsweise in Mengen nicht über 25 Gew.-%, insbesondere von 3 Gew.-% bis 20 Gew.-% und besonders bevorzugt in Mengen von 1 Gew.-% bis 15 Gew.-% eingesetzt. Unter diesen sind die kristallinen Natriumalumosilikate in Waschmittelqualität, insbesondere Zeolith A, Zeolith P sowie Zeolith MAP und gegebenenfalls Zeolith X, bevorzugt. Geeignete Alumosilikate weisen insbesondere keine Teilchen mit einer Korngröße über 30 µm auf und bestehen vorzugsweise zu wenigstens 80 Gew.-% aus Teilchen mit einer Größe unter 10 µm. Ihr Calciumbindevermögen liegt in der Regel im Bereich von 100 bis 200 mg CaO pro Gramm.

Als wasserlösliche Buildersubstanzen kommen vor allem Alkalicarbonate, Alkalihydrogencarbonate und/oder von Sesquicarbonaten bevorzugt. Insbesondere werden Natrium- und oder Kalium-Carbonate und -Hydrogencarbonate in den festen Textilwaschmitteln eingesetzt. Gegebenenfalls können auch geringe Mengen an Calciumcarbonaten in den festen Textilwaschmitteln enthalten sein.

Zusätzlich oder alternativ zum genannten wasserunlöslichen Alumosilikat und waeerlöslichen Alkalicarbonat können weitere wasserlösliche anorganische Buildermaterialien enthalten sein. Zu diesen gehören insbesondere die wasserlöslichen kristallinen und/oder amorphen Alkalisilikat-Builder. Derartige wasserlösliche anorganische Buildermaterialien sind in erfindungsgemäßen Mitteln vorzugsweise in Mengen von 1 Gew.-% bis 20 Gew.-%, insbesondere von 5 Gew.-% bis 15 Gew.-% enthalten. Die als Buildermaterialien brauchbaren Alkalisilikate weisen vorzugsweise ein molares Verhältnis von Alkalioxid zu SiO₂ unter 0,95, insbesondere von 1:1,1 bis 1:12 auf und können amorph oder kristallin vorliegen. Bevorzugte Alkalisilikate sind die Natriumsilikate, insbesondere die amorphen Natriumsilikate, mit einem molaren Verhältnis Na₂O:SiO₂ von 1:2 bis 1:2,8. Als kristalline Silikate, die allein oder im Gemisch mit amorphen Silikaten vorliegen können, werden vorzugsweise kristalline Schichtsilikate der allgemeinen Formel Na₂SiₓO₂ₓ₊₁ · y H₂O eingesetzt, in der x, das sogenannte Modul, eine Zahl von 1,9 bis 4 und y eine Zahl von 0 bis 20 ist und bevorzugte Werte für x 2, 3 oder 4 sind. Bevorzugte kristalline Schichtsilikate sind solche, bei denen x in der genannten allgemeinen Formel die Werte 2 oder 3 annimmt. Insbesondere sind sowohl ß- als auch δ-Natriumdisilikate (Na₂Si₂O₅ · y H₂O) bevorzugt. Auch aus amorphen Alkalisilikaten hergestellte, praktisch wasserfreie kristalline Alkalisilikate der obengenannten allgemeinen Formel, in der x eine Zahl von 1,9 bis 2,1 bedeutet, können in erfindungsgemäßen Mitteln eingesetzt werden. In einer weiteren bevorzugten Ausführungsform erfindungsgemäßer Mittel wird ein kristallines Natriumschichtsilikat mit einem Modul von 2 bis 3 eingesetzt, wie es aus Sand und Soda hergestellt werden kann. Natriumsilikate mit einem Modul im Bereich von 1,9 bis 3,5 werden in einer weiteren Ausführungsform erfindungsgemäßer Mittel eingesetzt. In einer bevorzugten Ausgestaltung erfindungsgemäßer Mittel setzt man ein granulares Additiv aus Alkalisilikat und Alkalicarbonat ein.

Geeignete Vergrauungsinhibitoren beziehungsweise soil-release-Wirkstoffe sind Celluloseether, wie Carboxymethylcellulose, Methylcellulose, Hydroxyalkylcellulosen und Cellulosemischether, wie Methylhydroxyethylcellulose, Methylhydroxypropylcellulose und Methyl-Carboxymethylcellulose. Vorzugsweise werden Natrium-Carboxymethylcellulose, Hydroxyporpylmethylcellulose und deren Gemische und gegebenengfalls deren Gemische mit Methylcellulose eingesetzt. Zu den üblicherweise eingesetzten Soil-release-Wirkstoffen gehören Copolyester, die Dicarbonsäureeinheiten, Alkylenglykoleinheiten und Polyalkylenglykoleinheiten enthalten. Der Anteil an Vergrauungsinhibitoren und/oder soil-release-Wirkstoffen in erfindungsgemäßen Mitteln liegt im allgemeinen nicht über 2 Gew.-% und beträgt vorzugsweise 0,5 Gew.-% bis 1,5 Gew.-%.

Als optische Aufheller für insbesondere Textilien aus Cellulosefasern (zum Beispiel Baumwolle) können beispielsweise Derivate der Diaminostilbendisulfonsäure beziehungsweise deren Alkalimetallsalze enthalten sein. Geeignet sind zum Beispiel Salze der 4,4'-Bis(2-anilino-4-morpholino-1,3,5-triazin-6-yl-amino)-stilben-2,2'-disulfonsäure oder gleichartig aufgebaute Verbindungen, die anstelle der Morpholinogruppe eine Diethanolaminogruppe, eine Methylaminogruppe oder eine 2-Methoxyethylaminogruppe tragen. Weiterhin können Aufheller vom Typ des substituierten 4,4'-Distyryl-diphenyl anwesend sein, zum Beispiel 4,4'-Bis-(4-chlor-3-sulfostyryl)-diphenyl. Auch Gemische von Aufhellern können verwendet werden. Für Polyamidfasern eignen sich besonders gut Aufheller vom Typ der 1,3-Diaryl-2-pyrazoline, beispielsweise 1-(p-Sulfoamoylphenyl)-3-(p-chlorphenyl)-2-pyrazolin sowie gleichartig aufgebaute Verbindungen. Der Gehalt des Mittels an optischen Aufhellern beziehungsweise Aufhellergemischen liegt im allgemeinen nicht über 1 Gew.-%, vorzugsweise 0,05 Gew.-% bis 0,5 Gew.-%. In einer bevorzugten Ausführungsform der Erfindung ist das Mittel frei von derartigen Wirkstoffen.

Zu den in den erfindungsgemäßen Mitteln einsetzbaren üblichen Schaumregulatoren gehören beispielsweise Polysiloxan-Kieselsäure-Gemische, wobei die darin enthaltene feinteilige Kieselsäure vorzugsweise silaniert oder anderweitig hydrophobiert ist. Die Polysiloxane können sowohl aus linearen Verbindungen wie auch aus vernetzten Polysiloxan-Harzen sowie aus deren Gemischen bestehen. Weitere Entschäumer sind Paraffinkohlenwasserstoffe, insbesondere Mikroparaffine und Paraffinwachse, deren Schmelzpunkt oberhalb 40 °C liegt, gesättigte Fettsäuren beziehungsweise Seifen mit insbesondere 20 bis 22 C-Atomen, zum Beispiel Natriumbehenat, und Alkalisalze von Phosphorsäuremono- und/oder -dialkylestern, in denen die Alkylketten jeweils 12 bis 22 C-Atome aufweisen. Unter diesen wird bevorzugt Natriummonoalkylphosphat und/oder -dialkylphosphat mit C₁₆- bis C₁₈-Alkylgruppen eingesetzt. Der Anteil der Schaumregulatoren kann vorzugsweise 0,2 Gew.-% bis 2 Gew.-% betragen.

Zur Einstellung des gewünschten pH-Werts können die erfindungsgemäßen Mittel system- und umweltverträgliche Säuren, insbesondere Citronensäure, Essigsäure, Weinsäure, Äpfelsäure, Milchsäure, Glykolsäure, Bernsteinsäure, Glutarsäure und/oder Adipinsäure, aber auch Mineralsäuren, insbesondere Schwefelsäure oder Alkalihydrogensulfate, oder Basen, insbesondere Ammonium- oder Alkalihydroxide, enthalten. Derartige pH-Regulatoren sind in den erfindungsgemäßen Mitteln vorzugsweise nicht über 10 Gew.-%, insbesondere von 0,5 Gew.-% bis 6 Gew.-%, enthalten.

Die erfindungsgemäßen Mittel liegen vorzugsweise als pulverförmige, granulare oder tablettenförmige Präparate vor, die in an sich bekannter Weise, beispielsweise durch Mischen, Granulieren, Walzenkompaktieren, Extrudieren und/oder durch Sprühtrocknung der thermisch belastbaren Komponenten und Zumischen der empfindlicheren Komponenten, zu denen insbesondere Enzyme und Bleichmittel sowie bleichaktivierende Systeme zu rechnen sind, hergestellt werden können. Die festen Textilwaschmittel können ein Schüttgewicht von 350 bis 950 g/l, nachverdichtete Mittel sogar darüber, aufweisen. Zur Herstellung erfindungsgemäßer Mittel mit erhöhtem Schüttgewicht, insbesondere im Bereich von 650 g/l bis 950 g/l, ist ein einen Extrusionschritt aufweisendes Verfahren bevorzugt.

Zur Herstellung von erfindungsgemäßen Textilwaschmitteln in Tablettenform geht man vorzugsweise derart vor, daß man alle Bestandteile oder verschiedene Additive in einem Mischer miteinander vermischt und das Gemisch mittels herkömmlicher Tablettenpressen, beispielsweise Exzenterpressen oder Rundläuferpressen, mit Preßdrucken im Bereich von 200 10⁵ Pa bis 1 500 10⁵ Pa verpresst. Man erhält so problemlos bruchfeste und dennoch unter Anwendungsbedingungen ausreichend schnell lösliche Tabletten mit Biegefestigkeit von normalerweise über 150 N. Vorzugsweise weist eine derart hergestellte Tablette ein Gewicht von 15 g bis 40 g, insbesondere von 20 g bis 30 g auf, bei einem Durchmesser von 35 mm bis 40 mm.

Einen weiteren Erfindungsgegenstand stellt ein Verfahren zur Reinigung von Textilien dar, wobei in mindestens einem Verfahrensschritt ein erfindungsgemäßes festes Textilwaschmittel angewendet wird. Hierunter fallen sowohl manuelle als auch maschinelle Verfahren, wobei maschinelle Verfahren aufgrund ihrer präziseren Steuerbarkeit, was beispielsweise die eingesetzten Mengen und Einwirkzeiten angeht, bevorzugt sind. Verfahren zur Reinigung von Textilien zeichnen sich im allgemeinen dadurch aus, dass in einem oder mehreren Verfahrensschritten verschiedene reinigungsaktive Substanzen auf das Reinigungsgut aufgebracht und nach der Einwirkzeit abgewaschen werden, oder dass das Reinigungsgut in sonstiger Weise mit einem Waschmittel oder einer Lösung dieses Mittels behandelt wird. Alle Sachverhalte, Gegenstände und Ausführungsformen, die für erfindungsgemäße textile Waschmittel beschrieben sind, sind auch auf diesen Erfindungsgegenstand anwendbar. Daher wird an dieser Stelle ausdrücklich auf die Offenbarung an entsprechender Stelle verwiesen mit dem Hinweis, dass diese Offenbarung auch für die vorstehenden erfindungsgemäßen Verfahren gilt.

Insbesondere werden in Verfahren, welche bei einer Temperatur zwischen 10°C und 50°C, bevorzugt zwischen 10°C und 40°C und besonders bevorzugt zwischen 20°C und 40°C durchgeführt werden, die Vorteile beim Einsatz der erfindungsgemäßen festen Textilwaschmittel gegenüber festen Textilwaschmittel mit herkömmlich eingesetzten Proteasen für den Verbraucher sichtbar.

Die in erfindungsgemäßen Mitteln eingesetzten Proteasen sind entsprechend der vorstehenden Ausführungen in festen Textilwaschmitteln sowie Verfahren zur Textilwäsche vorteilhaft einsetzbar. Sie können also vorteilhaft dazu verwendet werden, um in entsprechenden Mitteln und Verfahren eine proteolytische Aktivität bereitzustellen.

Einen weiteren Gegenstand der Erfindung bildet daher die Verwendung einer Protease,
(a1) die eine Aminosäuresequenz umfasst, die zu der in SEQ ID NO. 1 angegebenen Aminosäuresequenz zu mindestens 80% identisch ist und die an Position 99 in der Zählung gemäß SEQ ID NO. 1 die Aminosäure Glutaminsäure (E) oder Asparaginsäure (D) aufweist, oder
(a2) die eine Aminosäuresequenz umfasst, die zu der in SEQ ID NO. 1 angegebenen Aminosäuresequenz zu mindestens 80% identisch ist und die an Position 99 in der Zählung gemäß SEQ ID NO. 1 die Aminosäure Asparagin (N) oder Glutamin (Q) aufweist, zur Bereitstellung einer proteolytischen Aktivität in einem festen Textilwaschmittel, welches in 1 Gew.-%iger Lösung in entionisiertem Wasser bei 20 °C einen pH-Wert im Bereich von 4,0 bis unter 10 aufweist.

In einer weiteren Ausführungsform ist diese Verwendung dadurch gekennzeichnet, dass die Protease ferner in der Zählung gemäß SEQ ID NO. 1 mindestens eine der folgenden Aminosäuren aufweist:
(a) Threonin an Position 3 (3T),
(b) Isoleucin an Position 4 (4I),
(c) Alanin, Threonin oder Arginin an Position 61 (61A, 61T oder 61R),
(d) Asparaginsäure oder Glutaminsäure an Position 154 (154D oder 154E),
(e) Prolin an Position 188 (188P),
(f) Methionin an Position 193 (193M),
(g) Isoleucin an Position 199 (199I),
(h) Asparaginsäure, Glutaminsäure oder Glycin an Position 211 (211D, 211E oder 211G),
(i) Kombinationen der Aminosäuren (a) bis (h).
Alle Sachverhalte, Gegenstände und Ausführungsformen, die für das feste Textilwaschmittel beschrieben sind, sind auch auf die genannten Verwendungen anwendbar. Daher wird an dieser Stelle ausdrücklich auf die Offenbarung an entsprechender Stelle verwiesen mit dem Hinweis, dass diese Offenbarung auch für die vorstehenden erfindungsgemäßen Verwendungen gilt.
Besonders vorteilhaft ist auch die Verwendung eines erfindungsgemäßen festen Textilwaschmittels zur Entfernung von protease-sensitiven Anschmutzungen auf Textilien. Besonders bevorzugte Ausführungsformen stellen beispielsweise die Handwäsche, die manuelle Entfernung von Flecken von Textilien oder die Verwendung im Zusammenhang mit einem maschinellen Verfahren dar.

### Beispiele

Das Produkt Persil Megaperls® Color, wie es im April 2013 in Deutschland im Handel erhältlich war und welches eine Protease des Standes der Technik enthielt, die sehr gute Waschleistungen bei Temperaturen von 40 °C und darunter aufwies, diente als Vergleichsprodukt V zu einem erfindungsgemäßen festen Textilwaschmittel E, welches mit dem Vergleichsprodukt Persil Megaperls® Color mit Ausnahme der Protease identisch war. In dem erfindungsgemäßen festen Textilwaschmittel E wurde anstelle der herkömmlichen Protease eine Protease gemäß SEQ ID NO. 2 eingesetzt, die in Positionen 1 bis 98 und 100 bis 269 mit SEQ ID No. 2 übereinstimmte und die an Position 99 in der Zählung gemäß SEQ ID No. 1 die Aminosäure Glutaminsäure E aufwies. Beide Produkte E und V enthielten 0,76 Gew.-%, bezogen auf das jeweilige Mittel, der jeweiligen festen konfektionierten Enzym-Zubereitungsformen (Enzymgranulate). Beide Produkte enthielten weitere Enzyme in identischen Mengen. Beide Produkte E und V wurden auf ihre Leistungen gegenüber Protease-sensitiven Anschmutzungen getestet.

Hierzu wurden je 3,5 kg saubere Füllwäsche und 4 Schmutzbelastungstücher, wie sie im Handel erhältlich sind, mit jeweils 67,5 g E und V in einem 40 °C Normalprogramm in einer Miele-Waschmaschine und mit Wasser einer Wasserhärte von 15 bis 17 °d gewaschen und anschließend getrocknet (AISE-Testmethode). Es wurden folgende Schmutzbelastungstücher eingesetzt:

| | | |
|---|---|---|
| CFT CS01 | - | Blut auf Baumwolle |
| CFT C03 | - | Schokolade-Milch/Ruß auf Baumwolle |
| EMPA 112 | - | Kakao-Anschmutzung auf Baumwolle |
| EMPA 163 | - | Porridge-Anschmutzung auf Baumwolle |

Der Weißheitsgrad als Maß für die Reinigungsleistung, d.h. die Aufhellung der Anschmutzungen, wurde mit dem Spektrometer Minolta CM508d gemessen. Das Gerät war zuvor mit einem mitgelieferten Weißstandard auf 100 % kalibriert worden.

In der Tabelle 1 werden die Differenzen der Remissionswerte (RD) für E gegenüber V (d.h. die Remissionswerte der mit E gewaschenen Schmutzbelastungstücher waren um den in der Tabelle 1 angegebenen Differenzwert höher als für die mit V gewaschenen Schmutzbelastungstücher) als Mittelwerte aus 6 Bestimmungen sowie die Fehler bei der 6-fach Bestimmung (HSD) angegeben.

**Tabelle 1: Waschergebnisse (Differenz der Remissionswerte RD)**

| Schmutzbelastungstuch | RD (E gegenüber V) | HSD |
|---|---|---|
| CFT CS01 | 13,8 | 3,9 |
| CFT C03 | 7,9 | 1,7 |
| EMPA 112 | 4,8 | 1,5 |
| EMPA 163 | 3,4 | 2,1 |

Alle gemessenen Differenzwerte sind signifikant.

## Patentansprüche

1. Festes Textilwaschmittel umfassend
(a1) eine Protease, die eine Aminosäuresequenz umfasst, die zu der in SEQ ID NO. 1 angegebenen Aminosäuresequenz zu mindestens 80% identisch ist und die an Position 99 in der Zählung gemäß SEQ ID NO. 1 die Aminosäure Glutaminsäure (E) oder Asparaginsäure (D) aufweist, oder
(a2) eine Protease, die eine Aminosäuresequenz umfasst, die zu der in SEQ ID NO. 1 angegebenen Aminosäuresequenz zu mindestens 80% identisch ist und die an Position 99 in der Zählung gemäß SEQ ID NO. 1 die Aminosäure Asparagin (N) oder Glutamin (Q) aufweist,
wobei das feste Textilwaschmittel in 1 Gew.-%iger Lösung in entionisiertem Wasser bei 20 °C einen pH-Wert in einem Bereich von 4,0 bis unter 10 aufweist.

2. Festes Textilwaschmittel nach Anspruch 1, **dadurch gekennzeichnet, dass** die Protease ferner in der Zählung gemäß SEQ ID NO. 1 mindestens eine der folgenden Aminosäuren aufweist:
(a) Threonin an Position 3 (3T),
(b) Isoleucin an Position 4 (41),
(c) Alanin, Threonin oder Arginin an Position 61 (61A, 61T oder 61R),
(d) Asparaginsäure oder Glutaminsäure an Position 154 (154D oder 154E),
(e) Prolin an Position 188 (188P),
(f) Methionin an Position 193 (193M),
(g) Isoleucin an Position 199 (1991),
(h) Asparaginsäure, Glutaminsäure oder Glycin an Position 211 (211D, 211E oder 211G),
(i) Kombinationen der Aminosäuren (a) bis (h).

3. Festes Textilwaschmittel nach Anspruch 1 oder 2, **dadurch gekennzeichnet, dass** es mindestens ein zusätzliches Enzym derselben oder einer anderen Art enthält.

4. Festes Textilwaschmittel nach einem der vorherigen Ansprüche, **dadurch gekennzeichnet, dass** das mindestens eine weitere Enzym eine Protease, Amylase, Cellulase, Hemicellulase, Mannanase, Tannase, Xylanase, Xanthanase, Xyloglucanase, ß-Glucosidase, Pektinase, Carrageenase, Perhydrolase, Oxidase, Oxidoreduktase oder eine Lipase sowie deren Gemische umfasst.

5. Festes Textilwaschmittel nach einem der vorherigen Ansprüche, **dadurch gekennzeichnet, dass** es keine Bleiche, insbesondere keine anorganische oder organische Peroxidquelle enthält.

6. Festes Textilwaschmittel nach einem der vorherigen Ansprüche, **dadurch gekennzeichnet, dass** es einen pH-Wert (1 Gew.-%ige Lösung in entionisiertem Wasser bei 20 °C) von 6,5 bis 9,5 und insbesondere von 7,0 bis 9,0 aufweist.

7. Verfahren zur Reinigung von Textilien, **dadurch gekennzeichnet, dass** in mindestens einem Verfahrensschritt ein festes Textilwaschmittel nach einem der Ansprüche 1 bis 6 angewendet wird.

8. Verfahren nach Anspruch 7, **dadurch gekennzeichnet, dass** es bei einer Temperatur zwischen 10°C und 50°C, bevorzugt zwischen 10°C und 40°C und besonders bevorzugt zwischen 20°C und 40°C durchgeführt wird.

9. Verwendung einer Protease,
(a1) die eine Aminosäuresequenz umfasst, die zu der in SEQ ID NO. 1 angegebenen Aminosäuresequenz zu mindestens 80% identisch ist und die an Position 99 in der Zählung gemäß SEQ ID NO. 1 die Aminosäure Glutaminsäure (E) oder Asparaginsäure (D) aufweist, oder
(a2) die eine Aminosäuresequenz umfasst, die zu der in SEQ ID NO. 1 angegebenen Aminosäuresequenz zu mindestens 80% identisch ist und die an Position 99 in der Zählung gemäß SEQ ID NO. 1 die Aminosäure Asparagin (N) oder Glutamin (Q) aufweist, zur Bereitstellung einer proteolytischen Aktivität in einem festen Textilwaschmittel, welches in 1 Gew.-%iger Lösung in entionisiertem Wasser bei 20 °C einen pH-Wert im Bereich von 4 bis unter 10 aufweist.

10. Verwendung nach Anspruch 9, **dadurch gekennzeichnet, dass** die Protease ferner in der Zählung gemäß SEQ ID NO. 1 mindestens eine der folgenden Aminosäuren aufweist:
(a) Threonin an Position 3 (3T),
(b) Isoleucin an Position 4 (41),
(c) Alanin, Threonin oder Arginin an Position 61 (61A, 61T oder 61R),
(d) Asparaginsäure oder Glutaminsäure an Position 154 (154D oder 154E),
(e) Prolin an Position 188 (188P),
(f) Methionin an Position 193 (193M),
(g) Isoleucin an Position 199 (1991),
(h) Asparaginsäure, Glutaminsäure oder Glycin an Position 211 (211D, 211E oder 211G),
(i) Kombinationen der Aminosäuren (a) bis (h).

## Claims

1. A solid laundry detergent, comprising:
(a1) a protease comprising an amino acid sequence that is at least 80% identical to the amino acid sequence set forth in SEQ ID NO. 1 and contains at position 99 in the numbering according to SEQ ID NO. 1 the amino acid glutamic acid (E) or aspartic acid (D); or
(a2) a protease comprising an amino acid sequence that is at least 80% identical to the amino acid sequence set forth in SEQ ID NO. 1 and contains at position 99 in the numbering according to SEQ ID NO. 1 the amino acid asparagine (N) or glutamine (Q),
wherein the solid laundry detergent in a 1 wt.% solution in deionized water at 20°C has a pH value in a range from 4.0 to less than 10.

2. The solid laundry detergent according to claim 1, **characterized in that** the protease, in the numbering according to SEQ ID NO. 1, further comprises at least one of the following amino acids:
(a) threonine at position 3 (3T);
(b) isoleucine at position 4 (4I);
(c) alanine, threonine or arginine at position 61 (61A, 61T or 61R);
(d) aspartic acid or glutamic acid at position 154 (154D or 154E);
(e) proline at position 188 (188P);
(f) methionine at position 193 (193M);
(g) isoleucine at position 199 (199I);
(h) aspartic acid, glutamic acid or glycine at position 211 (211D, 211E or 211G),
(i) combinations of amino acids (a) to (h).

3. The solid laundry detergent according to claim 1 or 2, **characterized by** comprising at least one additional enzyme of the same or of another type.

4. The solid laundry detergent according to any one of the preceding claims, **characterized in that** the at least one further enzyme comprises a protease, an amylase, a cellulase, hemicellulase, mannanase, tannase, xylanase, xanthanase, xyloglucanase, β-glucosidase, pectinase, carrageenase, perhydrolase, oxidase, oxidoreductase or a lipase, or the mixtures thereof.

5. The solid laundry detergent according to any one of the preceding claims, **characterized in that** it comprises no bleach, in particular no inorganic or organic peroxide source.

6. The solid laundry detergent according to any one of the preceding claims, **characterized by** having a pH value (1 wt.% solution in deionized water at 20°C) of 6.5 to 9.5, and in particular of 7.0 to 9.0.

7. A method for cleaning textiles, **characterized in that** in at least one method step a solid laundry detergent according to any one of claims 1 to 6 is used.

8. The method according to claim 7, **characterized in that** it is carried out at a temperature between 10°C and 50°C, preferably between 10°C and 40°C, and particularly preferably between 20°C and 40°C.

9. Use of a protease,
(a1) which comprises an amino acid sequence that is at least 80% identical to the amino acid sequence set forth in SEQ ID NO. 1 and contains the amino acid glutamic acid (E) or aspartic acid (D) at position 99 in the numbering according to SEQ ID NO. 1; or
(a2) which comprises an amino acid sequence that is at least 80% identical to the amino acid sequence set forth in SEQ ID NO. 1 and contains the amino acid asparagine (N) or glutamine (Q) at position 99 in the numbering according to SEQ ID NO. 1;
for providing a proteolytic activity in a solid laundry detergent that in a 1 wt.% solution in deionized water at 20°C has a pH value in the range from 4 to less than 10.

10. The use according to claim 9, **characterized in that** the protease, in the numbering according to SEQ ID NO. 1, further comprises at least one of the following amino acids:
(a) threonine at position 3 (3T);
(b) isoleucine at position 4 (4I);
(c) alanine, threonine or arginine at position 61 (61A, 61T or 61R);
(d) aspartic acid or glutamic acid at position 154 (154D or 154E);
(e) proline at position 188 (188P);
(f) methionine at position 193 (193M);
(g) isoleucine at position 199 (199I);
(h) aspartic acid, glutamic acid or glycine at position 211 (211D, 211E or 211G),
(i) combinations of amino acids (a) to (h).

## Revendications

1. Détergent solide pour textiles comprenant
(a1) une protéase qui comporte une séquence d'acides aminés qui est identique à au moins 80% à la séquence d'acides aminés de SEQ ID N° 1 et qui comporte à la position 99 dans le comptage selon SEQ ID N° 1 l'acide aminé acide glutamique (E) ou acide aspartique (D), ou
(a2) une protéase qui comporte une séquence d'acides aminés qui est identique à au moins 80% à la séquence d'acides aminés de SEQ ID N° 1 et qui comporte à la position 99 dans le comptage selon SEQ ID N° 1 l'acide aminé asparagine (N) ou glutamine (Q)
le détergent solide pour textiles ayant un pH dans la gamme de 4,0 à 10 dans une solution à 1% en poids dans de l'eau dé-ionisée à 20°C.

2. Détergent solide pour textiles selon la revendication 1, dans lequel la protéase comporte en outre dans le comptage selon SEQ ID N° 1 au moins l'un des acides aminés suivants :
(a) la thréonine à la position 3 (3T),
(b) l'isoleucine à la position 4 (4I),
(c) l'alanine, la thréonine ou l'arginine à la position 61 (61A, 61T ou 61R),
(d) l'acide aspartique ou l'acide glutamique à la position 154 (154D ou 154E)
(e) la proline à la position 188 (188P),
(f) la méthionine à la position 193 (193M),
(g) l'isoleucine à la position 199 (199I),
(h) l'acide aspartique, l'acide glutamique ou la glycine à la position 211 (211D, 211E ou 211G),
(i) combinaisons des acides aminés (a) à (h).

3. Détergent solide pour textiles selon la revendication 1 ou 2, **caractérisé en ce qu'**il contient au moins une enzyme supplémentaire de même type ou de type différent.

4. Détergent solide pour textiles selon l'une des revendications précédentes, **caractérisé en ce que** l'au moins une autre enzyme est une protéase, une amylase, une cellulase, une hémicellulase, une mannanase, une tannase, une xylanase, une xanthanase, une xyloglucanase, une β-glucosidase, une pectinase, une carraghénase, une perhydrolase, une oxydase, une oxydoréductase ou une lipase ainsi que leurs mélanges.

5. Détergent solide pour textiles selon l'une des revendications précédentes, **caractérisé en ce qu'**il ne contient pas d'agent de blanchiment, en particulier de source de peroxyde minéral ou organique.

6. Détergent solide pour textiles selon l'une des revendications précédentes, **caractérisé en ce qu'**il elle présente un pH (solution à 1% en poids dans de l'eau dé-ionisée à 20°C) de 6,5 à 9,5 et en particulier de 7,0 à 9,0.

7. Procédé de nettoyage de textiles, **caractérisé en ce que**, dans au moins une étape de procédé, on utilise un détergent solide pour textiles selon l'une des revendications 1 à 6.

8. Procédé selon la revendication 7, **caractérisé en ce qu'**il est mis en oeuvre à une température comprise entre 10°C et 50°C, de préférence entre 10°C et 40°C et en particulier entre 20°C et 40°C.

9. Utilisation d'une protéase,
(a1) qui comporte une séquence d'acides aminés qui est identique à au moins 80% à la séquence d'acides aminés de SEQ ID N° 1 et qui comporte à la position 99 dans le comptage selon SEQ ID N° 1 l'acide aminé acide glutamique (E) ou acide aspartique (D), ou
(a2) qui comporte une séquence d'acides aminés qui est identique à au moins 80% à la séquence d'acides aminés de SEQ ID N° 1 et qui comporte à la position 99 dans le comptage selon SEQ ID N° 1 l'acide aminé asparagine (N) ou glutamine (Q)
le détergent solide pour textiles ayant un pH dans la gamme de 4,0 à 10 dans une solution à 1% en poids dans de l'eau dé-ionisée à 20°C.

10. Utilisation selon la revendication 9, **caractérisée en ce que** la protéase comporte en outre dans le comptage selon SEQ ID N° 1 au moins l'un des acides aminés suivants :
(a) la thréonine à la position 3 (3T),
(b) l'isoleucine à la position 4 (4I),
(c) l'alanine, la thréonine ou l'arginine à la position 61 (61A, 61T ou 61R),
(d) l'acide aspartique ou l'acide glutamique à la position 154 (154D ou 154E)
(e) la proline à la position 188 (188P),
(f) la méthionine à la position 193 (193M),
(g) l'isoleucine à la position 199 (199I),
(h) l'acide aspartique, l'acide glutamique ou la glycine à la position 211 (211D, 211E ou 211G),
(i) combinaisons des acides aminés (a) à (h).
